Europäisches Patentamt

European Patent Office

Office européen des brevets

(19)

(11) Veröffentlichungsnummer : **0 472 547 B1**

(12) **EUROPÄISCHE PATENTSCHRIFT**

(45) Veröffentlichungstag der Patentschrift :
09.12.92 Patentblatt 92/50

(51) Int. Cl.$^5$ : **A61M 25/06**

(21) Anmeldenummer : **90906888.4**

(22) Anmeldetag : **07.05.90**

(86) Internationale Anmeldenummer :
**PCT/DE90/00328**

(87) Internationale Veröffentlichungsnummer :
**WO 90/13330 15.11.90 Gazette 90/26**

(54) **PUNKTIONSINSTRUMENT.**

(30) Priorität : **05.05.89 DE 3915215**

(43) Veröffentlichungstag der Anmeldung :
**04.03.92 Patentblatt 92/10**

(45) Bekanntmachung des Hinweises auf die
Patenterteilung :
**09.12.92 Patentblatt 92/50**

(84) Benannte Vertragsstaaten :
**AT BE CH DE DK ES FR GB IT LI LU NL SE**

(56) Entgegenhaltungen :
**EP-A- 0 064 212**
**US-A-33 996 74**
**US-A-36 772 43**

(73) Patentinhaber : **MÖHRING, Klaus**
**Uferstrasse 40**
**W-6900 Heidelberg (DE)**
Patentinhaber : **MAGASI, Josef**
**Wendelinusstrasse 8**
**W-6902 Sandhausen (DE)**

(72) Erfinder : **MÖHRING, Klaus**
**Uferstrasse 40**
**W-6900 Heidelberg (DE)**
Erfinder : **MAGASI, Josef**
**Wendelinusstrasse 8**
**W-6902 Sandhausen (DE)**

(74) Vertreter : **Maikowski, Michael, Dipl.-Ing. Dr. et al**
**Patentanwälte Maikowski & Ninnemann**
**Xantener Strasse 10**
**W-1000 Berlin 15 (DE)**

**Beschreibung**

Die Erfindung betrifft ein Gerät nach dem Oberbegriff des Patentanspruchs 1.

Aus der DE-C 30 48 774 ist eine zweiteilige Katheterisierungsvorrichtung bekannt, bei der das Punktionsrohr einen Handgriff aufweist. In dieses Punktionsrohr wird vor dem Punktieren eine rohrförmige Stahlkanüle eingesetzt, die an ihrem vorderen Ende eine abgeschrägte Schneidkante und an ihrem rückwärtigen Ende einen Handgriff aufweist. Bei dieser bekannten Katheterisierungsvorrichtung sind zwei im axialen Abstand voneinander angeordnete Handgriffe vorgesehen. Beim Durchbohren der Haut wirkt diese bekannte Katheterisierungsvorrichtung wie der ringförmige Stirnrand einer zylindrischen Stanze. Trotz der abgeschrägten Schneidkante stanzt das bekannte Gerät beim Eindrücken dieser Katheterisierungsvorrichtung in die Haut, bzw. in das Körpergewebe Haut- und Gewebepropfen aus der Körperpartie heraus, die in den Hohlraum der Kanüle hineingedrückt werden. Dem Eindrücken der kreisringförmigen Stirnränder dieser Vorrichtung in menschliches oder tierisches Gewebe, insbesondere in Muskelgewebe, wirkt ein hoher Widerstand entgegen, der durch entsprechende Kräfte überwunden werden muß. Die kreisringförmige Stanzkante erhöht dabei erheblich die Gefahr einer Verletzung von Gefäßen. Es erfolgt zwangsläufig ein Transport herausgestanzter Haut- oder Gewebeteile in andere Körperregionen und dieser Transport kann unerwünschte Folgen, wie beispielsweise Infektionen, nach sich ziehen. Mit diesen bekannten Geräten kann es bei der suprapubischen Harnableitung zu einer nicht unerheblichen Traumatisierung der gefüllten Blasenwand, zum Teil auch zum Ausstanzen des, die Blase umgebenden Gewebes und zu größeren Blutungen in der Umgebung der Blase oder in das Blasenlumen kommen.

Im Merkblatt 51-240-03, Mai 1976 der Firma DOW CORNING EUROPE, Brüssel wird ein suprapubisches Drainage-System nach Reif beschrieben, das einen Trokar mit pyramiden-förmiger Spitze in einer zylindrischen Kanüle aufweist. Beim Einführen dieses Gerätes ist ein hoher Widerstand zu überwinden, da dessen Spitze stumpf in die Haut eindringt und das Gewebe verdrängt.

Der Erfindung liegt die Aufgabe zugrunde, ein Punktionsinstrument zu schaffen, mit dem das Herausstanzen von Körper- oder Organteilen während der Punktion weitgehend vermieden wird.

Erfindungsgemäß wird diese Aufgabe durch die technische Lehre des Patentanspruchs 1 gelöst.

In vorteilhafter Weise ist das in das Punktionsrohr einpaßbare Punkturinstrument eine teilzylindrisch gewölbte rinnenförmige Stichinzisions-Lanze. Diese rinnenförmige Stichinzisions-Lanze ist kein vollzylindrischer Bauteil und weist keine kreisringförmige Stirnwandung auf, die pfropfenartige Körperteile herausstanzt. Mit Vorteil ist die Schneidkante der Stichinzisions-Lanze derart gestaltet, daß eine optimale Annäherung an einen geradlinigen Schnitt erreicht wird. Am Ende des Punktionsrohres ist ein Abschnitt eines Rohrkrümmers in einer speziellen Weise ausgebildet. Das Außenradiusbogenende dieses Rohrkrümmerabschnittes erstreckt sich bis zur Rinneninnenseite der eingesetzten Stichinzisions-Lanze und liegt gegen diese an. Dadurch erstreckt sich der Öffnungsrand dieses Rohrkrümmerabschnittes über die Rinnenseitenkanten der Stichinzisions-Lanze hinweg in die Rinneninnenseite. Dieser Öffnungsrand bildet eine Raumkurve, die in zwei zueinander senkrechten Richtungen gekrümmt ist und aus zwei zur Trennebene des Punktionsrohres spiegelbildlichen Kurvenabschnitten besteht. Durch diese Gestaltung ist das untere Ende des Punktionsrohres nicht, wie bei den bekannten Geräten, ein offener und damit stanzender Kreisring, sondern bildet einen in der Form eines Schiffsbuges gegen die Stichinzisions-Lanze anliegenden Abschluß. Dieser schiffsbugartige Abschluß, der sich unmittelbar an die Stichinzisions-Lanze anschließt, verdrängt beim Einstechen des Gerätes in den Körper das Gewebe und übt selbst kaum Schnittwirkungen aus. Ein Stanzen wird weitgehend ausgeschlossen; die von der Stichinzisions-Lanze geschnittenen Gewebeteile werden lediglich verdrängt. Neben der Vermeidung unerwünschter Stanz- oder Schnittwirkungen wird der Eindrängungswiderstand erheblich verringert. Die Ausbildung der Gerätespitze gleicht optimal der einer starken Nadel oder Spitze.

Die erfindungsgemäße Ausbildung des Punktionsinstrumentes bietet weiter den Vorteil, daß ultraschallgesteuerte Punktionen erleichtert werden. Die Spitzen der bekannten Punktionsinstrumente sind wenig echogen, d.h. ergeben kein ausreichendes Bild, um die Punktion mittels Ultraschall zu steuern. Die Echomuster sind beim Erfindungsgegenstand gut, teilweise sogar ausgezeichnet, ausgebildet.

Die teilzylindrisch gewölbte, rinnenförmige Gestaltung der Stichinzisions-Lanze ermöglicht, die Schneidkante als Lanzettenschliff mit gewünschtem Winkel auszubilden.

Eine ergonomische optimale Gestaltung des Gerätes wird dadurch erreicht, daß der Handgriff der Stichinzisions-Lanze eine, zur Oberseite des Handgriffs des Punktionsrohres komplementär ausgebildete Unterseite und zwei Rastzapfen sowie eine Aufnahmenut aufweist. Durch diese Gestaltung können die Handgriffteile zu einem integralen Handgriff vereinigt werden. In die Aufnahmenut wird der einzuführende, langgestreckte Gegenstand, zum Beispiel ein Katheter, eingelegt. Ein axiales Herausführen dieses lang gestreckten Gegenstandes aus dem Gerät wird vermieden, so daß die Handhabung des Gerätes wesentlich erleichtert wird. Zur Verbesserung der Handhabung ist ferner der Handgriff der Stichinzisions-Lanze auf der Oberseite mit Rillen versehen, insbesondere in dem Bereich, in dem der Daumen aufgelegt werden kann.

Bei einer besonders vorteilhaften Ausgestaltung des erfindungsgemäßen Punktionsinstrumentes ist das Punktionsrohr teilbar und kann daher zum Beispiel nach dem Legen des Katheters aus dem Körper herausgezogen und anschließend durch einfaches Auseinanderziehen beider Teile entfernt werden.

Jede Hälfte des teilbaren Punktionsrohres kann einen, mit einer Rastnut ausgerüsteten koplementären Abschnitt eines Handgriffes tragen. Durch diese Ausgestaltung können die beiden Rohrabschnitte des teilbaren Punktionsrohres miteinander verriegelt werden.

Ein Ausführungsbeispiel der Erfindung soll unter Bezugnahme auf die Figuren der Zeichnung erläutert werden.

Es zeigen:
Fig. 1 eine schematische perspektivische Ansicht eines Punktionsinstrumentes
Fig. 2 eine vergrößerte Detailansicht des unteren Endes des Punktionsinstrumentes,
Fig.3 + Fig.4 perspektivische Ansichten des oberen Endes des Punktionsinstrumentes in zwei verschiedenen Betriebsstellungen.

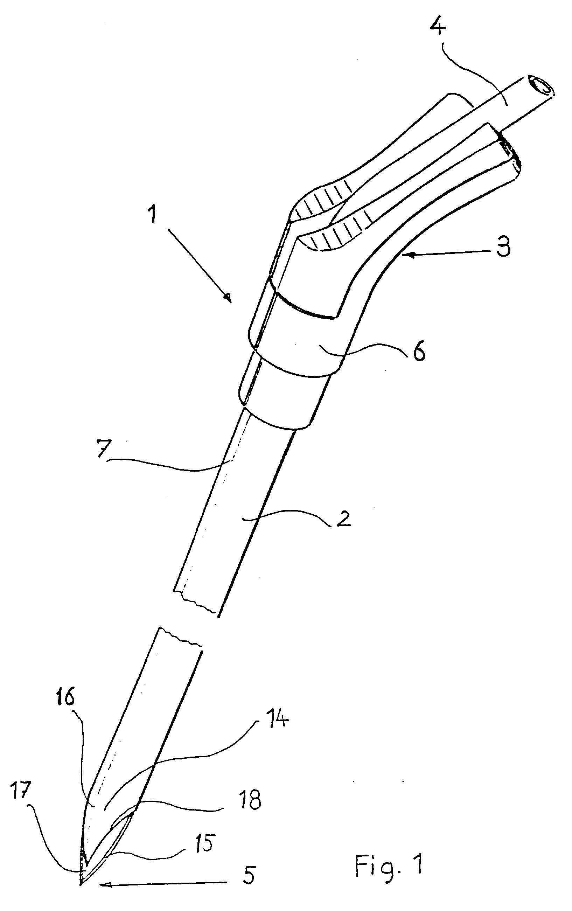

Das in Fig. 1 dargestellte Punktionsinstrument 1 weist ein geteiltes Punktionsrohr 2 auf. Die aneinanderstoßenden Kanten des zusammengesetzten Punktionsrohres 2 sind bei 7 dargestellt. Durch diese Kanten 7 hindurch erstreckt sich die Trennebene des Punktionsrohres 2.

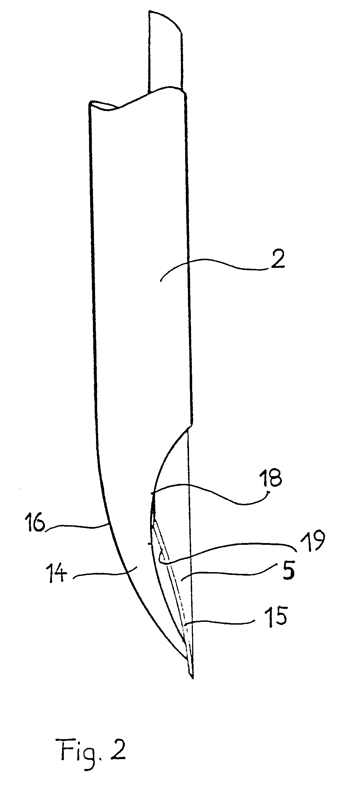

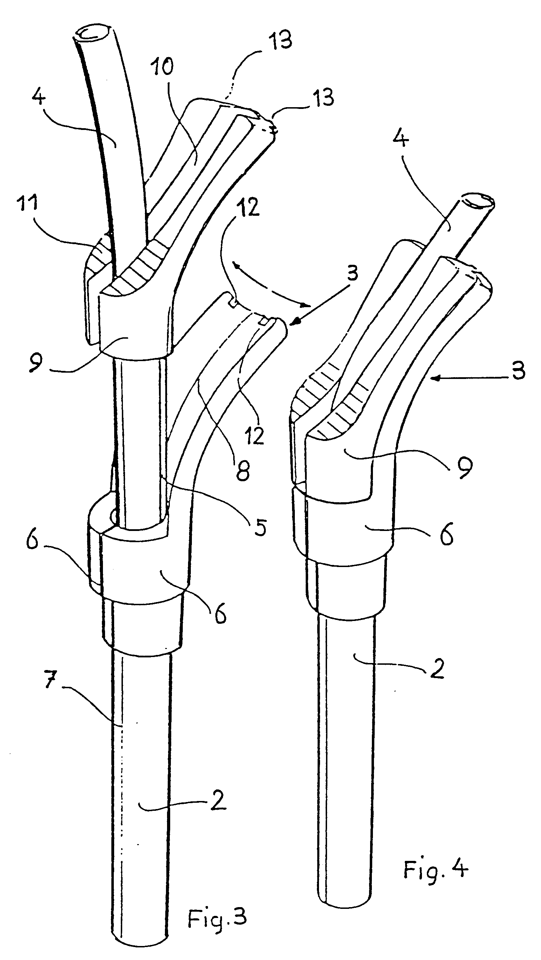

Wie Fig. 3 zeigt, ist in dieses Punktionsrohr 2 eine teilzylindrisch gewölbte, rinnenförmige Stichinzisions-Lanze 5 eingesetzt. Der Außenumfang dieser rinnenförmigen Stichinzisions-Lanze 5 ist an den Innenumfang des Punktionsrohres 2 angepaßt. Im eingesetzten Zustand, der in den Fig. 1, 2 und 4 dargestellt ist, erstreckt sich, wie Fig. 1 und 2 zeigen, das untere Ende der Stichinzisions-Lanze 5 aus dem Punktionsrohr 2 heraus. Am unteren Ende der Stichinzisions-Lanze 5 ist ein Lanzettenschliff 15 ausgebildet.

Wie die Fig. 1 und 2 zeigen, ist am unteren Ende des geteilten Punktionsrohres 2 ein Abschnitt 14 eines Rohrkrümmers ausgebildet. Der Rohrkrümmer ist durch Querschleifen, wie dargestellt, abgestochen. Durch das Schleifen wird ein Öffnungsrand 18 erzeugt, der eine Raumkurve bildet, die in zwei zueinander senkrechten Richtungen gekrümmt ist. Dieser Öffnungsrand 18 geht, wie der Fig. 2 zu entnehmen ist, oben von einer Stelle aus, die im Bereich der aneinander anstoßenden Kanten 7 des geteilten Punktionsrohres 2 liegt. Der Öffnungsrand 18 ist derart gestaltet, daß sich der in den Fig. 1 und 2 dargestellte Außenradiusbogen 16 bis zur Rinneninnenseite 17 erstreckt und an dieser anliegt. Der Öffnungsrand 18 erstreckt sich über beide Rinnenseitenkanten 19 hinweg zur Rinneninnenseite 17 hin, so daß, wie Fig. 1 zeigt, das untere Ende des Punktionsrohres 2 die Form eines keilförmig oder spitz zulaufenden Verdrängerkörpers hat, dessen Spitze gegen die Rinneninnenseite 17 der teilzylindrisch gewölbten, rinnenförmigen Stichinzisions-Lanze 5 anliegt.

Wie die Fig. 3 und 4 zeigen, weist jede Hälfte des Punktionsrohres 2 einen komplementären Abschnitt 6 eines Handgriffs 3 auf. Deren Stoßkante im zusammengebauten Zustand ist in Fig. 3 mit 8 gekennzeichnet. Diese komplementären Abschnitte 6 sind an der Außenkante mit Rastnuten 12 versehen.

Am Ende der Stichinzisions-Lanze 5 ist ein Handgriff 9 ausgebildet, der eine Aufnahmenut 10 und an seinem Ende Rastnasen 13 aufweist. Im zusammengesetzten Zustand, der in Fig. 4 dargestellt ist, greifen die Rastnasen 13 in die Rastnuten 12 der komplementären Abschnitte 6 des Handgriffs 3 ein und verriegeln somit die Bauteile.

In der Fig. 1, 3 und 4 ist schematisch ein Katheter 4 dargestellt, der mit dem Punktionsinstrument 1 in den Körper eingeführt werden soll. In die Aufnahmenut 10 (Fig. 3) des Handgriffs 9 kann, wie Fig. 4 zeigt, der Katheter 4 eingelegt werden. Wenn die Hand des Benutzers den, in Fig. 4 dargestellten, aus den Griffen 3 und 9 gebildeten Handhabungsgriff erfaßt, wird die Manipulation durch einen axial sich erstreckenden Katheter 4 nicht gestört. Eine Riffelung 11 an der Oberseite des Handgriffes 3 erleichtert die Manipulation.

## Patentansprüche

1. Punktionsinstrument, bestehend aus einem Punktionsrohr und einem angepaßten Punkturinstrument
dadurch gekennzeichnet, daß
das Punktur-Instrument eine teilzylindrisch gewölbte, rinnenförmige Stichinzisions-Lanze (5) ist,
das Punktionsrohr (2), das einen Handgriff (3) aufweist, am Ende als ein Abschnitt (14) eines Rohrkrümmers ausgebildet ist,
dessen Außenradiusbogenende (16) an der Rinneninnenseite (17) der eingesetzten Stichinzisions-Lanze (5) anliegt.

2. Punktionsinstrument nach Anspruch 1,
dadurch gekennezeichnet, daß
der Handgriff (9) der Stichinzisions-Lanze (5) eine zur Oberseite des Handgriffs (3) des Punktionsrohres (2) komplementär ausgebildete Unterseite und zwei Rastzapfen (13) sowie eine Aufnahmenut (10) für einen Katheter aufweist.

3. Punktionsinstrument nach Anspruch 2,
dadurch gekennzeichnet, daß

der Handgriff (9) der Stichinzisions-Lanze (5) auf der Oberseite Rillen (11) aufweist.

4. Punktionsinstrument nach einem der Ansprüche 1 bis 3,
   dadurch gekennzeichnet, daß
   es über seine gesamte Länge teilbar ist.

5. Punktionsrohr nach Anspruch 4
   dadurch gekennzeichnet, daß
   jede Hälfte des teilbaren Punktionsrohres (2) einen, mit einer Rastnut (12) ausgerüsteten komplementären Abschnitt (6) eines Handgriffs (3) trägt.

## Claims

1. Puncturing instrument, comprising a puncturing tube and a conforming puncture instrument, characterised in that the puncture instrument is a partially cylindrically curved channel-like prick incision lance (5) and the puncturing tube (2) which has a handle (3) is designed at the end as a section (14) of a pipe bend whose outer radius curved end (16) adjoins the channel inside (17) of the inserted prick incision lance (5).

2. Puncturing instrument according to claim 1, characterised in that the handle (9) of the prick incision lance (5) has an underside which complements the upper side of the handle (3) of the puncturing tube (2) and also has two detent pins (13) and a socket groove (10) for a catheter.

3. Puncturing instrument according to claim 2, characterised in that the handle (9) of the prick incision lance (5) has grooves (11) on the upper side.

4. Puncturing instrument according to one of claims 1 to 3 characterised in that it can be divided over its entire length.

5. Puncturing tube according to claim 4 characterised in that each half of the divisible puncturing tube (2) supports a complementary section (6) of a handle (3) fitted with a detent groove (12).

## Revendications

1. Instrument de ponction constitué par un tube de ponction et un instrument de perçage adapté,
   caractérisé en ce que
   l'instrument de perçage est constitué par une lance d'incision par piqûre (5) en forme de canalisation de forme courbe et partiellement cylindrique,

le tube de ponction (2) qui comprend une poignée (3) est constitué à son extrémité par une section (14) en forme de coude tubulaire,
   dont l'extrémité (16) du coude à rayon externe s'applique contre le côté interne (17) de la canalisation de la lance d'incision par piqûre (5) qui y est introduite.

2. Instrument de ponction selon la revendication 1,
   caractérisé en ce que la poignée (9) de la lance d'incision par piqûre (5) comprend un côté inférieur de constitution complémentaire de celle du côté supérieur de la poignée (3) du tube de ponction (2) et deux saillies de verrouillage (13) ainsi qu'une gorge de réception (10) pour un cathéter.

3. Instrument de ponction selon la revendication 2,
   caractérisé en ce que la poignée (9) de la lance d'incision par piqûre (5) comprend des stries (11) sur son côté supérieur.

4. Instrument de ponction selon l'une quelconque des revendications 1 à 3,
   caractérisé en ce qu'il peut être subdivisé sur la totalité de sa longueur.

5. Instrument de ponction selon la revendication 4,
   caractérisé en ce que chaque moitié du tube de ponction divisible (2) supporte une section complémentaire (6) d'une poignée (3) qui est équipée d'une encoche de verrouillage (12).

EP 0 472 547 B1

Fig. 1

Fig. 2

Fig. 3

Fig. 4